# EUROPÄISCHE PATENTSCHRIFT

(11) **EP 0 812 576 B1**
(45) Veröffentlichungstag und Bekanntmachung des Hinweises auf die Patenterteilung: **23.10.2002**
(21) Anmeldenummer: 97109683.9
(22) Anmeldetag: 13.06.1997
(51) Int. Cl.: A61B 18/12

(54) **Elektrochirurgischer Hochfrequenz-Generator**
High frequency generator for electrosurgery
Générateur de haute fréquence pour électrochirurgie

(30) Priorität: 14.06.1996 DE 19623840
(43) Veröffentlichungstag der Anmeldung: 17.12.1997
(73) Patentinhaber: Berchtold Holding GmbH, 78532 Tuttlingen (DE)
(72) Erfinder: Schilling, Bertram, 78194 Mauenheim (DE); Tockweiler, Udo, 78194 Immendingen (DE)
(74) Vertreter: Manitz, Gerhart, Dr.

(56) Entgegenhaltungen:
- WO-A-93/03677
- DE-A- 3 120 102
- FR-A- 2 502 935
- GB-A- 897 961

## Beschreibung

Die Erfindung betrifft einen elektrochirurgischen Hochfrequenz-Generator nach dem Oberbegriff des Anspruchs 1 (vgl. WO-A-93/03677).

Die Anwendung von Hochfrequenzenergie für Schneide- und Koagulationseffekte an menschlichen oder tierischen Körpern erforderte eine applikationsorientierte Leistungsabgabe abhängig vom verwendeten Instrument und vom Übergangswiderstand zum Gewebe. Die schließlich erzielte elektrochirurgische Wirkung hängt von vielen verschiedenen Parametern ab, wie z.B. der Generatorstabilität, der Ausgangsspannung, dem Ausgangsstrom, der Kabellänge zum Instrument, der Kapazität des Kabels, der Form und Oberfläche des Instruments, dem Gewebewiderstand usw.

Um die verschiedenen Einflußgrößen wenigstens in gewissem Umfang zu berücksichtigen, wurde bei älteren Geräten durch geeignete Dimensionierung des Ausgangsübertragers die Übertragungsfunktion "Spannung über Impedanz" oder "Strom über Impedanz" bestimmt. Demgegenüber war es ein Fortschritt, den Ausgangsstrom (DE-OS 31 20 102) oder mit Hilfe von Strom-/Spannungssensoren die Leistung konstant zu halten (DE-OS 25 04 280, DE-OS 27 10 752). Eine weitere Verbesserung der Ergebnisse wurde dadurch erreicht, daß aus den Momentanwerten von Strom und Spannung ein Impedanzwert errechnet und dieser zur Regelung verwendet wurde (DE-OS 31 20 102). Nachteilig hierbei ist, daß bei den üblichen Arbeitsfrequenzen von Hochfrequenz-Generatoren (300 kHz bis 1 MHz) sich die Leitungskapazitäten der Hin- und Rückleitung stark auswirken, so daß im hochohmigen Bereich nicht eindeutig festgestellt werden kann, wie groß die Gewebeimpedanz tatsächlich ist.

Eine weitere Ausführung von Hochfrequenz-Generatoren arbeitet mit einem Sensor für die Erkennung des Vorhandenseins von Funken oder Lichtbögen an derjenigen Stelle, wo ein Schneideffekt erzielt werden soll (EP-OS 0 219 568). Nachteilig an dieser Anordnung ist, daß, solange der Lichtbogen nicht zündet, die Spannung sehr stark ansteigt. Zur Vermeidung von Gefahren für die Patienten und die Bedienungsperson müssen daher Spannungsbegrenzungseinrichtungen abhängig von der vorzugebenden Leistungsstufe das zu starke Hochfahren der Spannung verhindern.

Weiter ist es bereits bekannt (GB-PS 897 961), in den Hochfrequenzausgangskreis eines Hochfrequenz-Generators einen Gleichstrom einzuspeisen, mittels dessen der Widerstand zwischen Aktivelektrode und Neutralelektrode festgestellt und zum Einschalten der Hochfrequenz verwendet wird.

Schließlich ist es schon bekannt (DE-OS 11 39 927), die richtige Anlage einer Neutralelektrode am Patientenkörper durch Aufteilung der Neutralelektrode in zwei voneinander isolierte Bereiche und Anlegen einer Gleichspannung an diese beiden Elektrodenbereiche festzustellen.

Das Ziel der vorliegenden Erfindung besteht darin, einen Hochfrequenz-Generator der eingangs genannten Gattung zu schaffen, der einfach bedienbar ist und dabei gleichzeitig die beim Schneiden eingesetzte Leistung optimiert.

Zur Lösung dieser Aufgabe sind die Merkmale des kennzeichnenden Teils des Anspruches 1 vorgesehen.

Vorteilhafte Weiterbildungen der Erfindung entnimmt man den Unteransprüchen.

Der erfindungsgemäße Gewebeimpedanzsensor soll in erster Linie feststellen, ob ein ausreichender Kontakt der Aktiv- und Neutralelektrode mit dem Gewebe vorliegt. Insofern kann der Gewebeimpedanzsensor auch als Gewebekontaktsensor bezeichnet werden. Bevorzugt beschränkt sich die Funktion des Gewebeimpedanz- bzw. -kontaktsensors aber nicht darauf, nur festzustellen, ob ein Gewebekontakt vorhanden ist oder nicht, sondern erstreckt sich auch auf eine Feststellung des Grades des Kontaktes bzw. des Ausmaßes der Stromleitfähigkeit des Gewebes, so daß eine besonders feinfühlige Regelung in Abhängigkeit von der momentanten Gewebeimpedanz möglich ist.

Erfindungsgemäß wird ein üblicher Hochfrequenz-Generator, der insbesondere bereits Hochfrequenzstrom-, Hochfrequenzspannungs- und Lichtbogenerkennungssensoren aufweist, durch einen Gewebeimpedanz-Erkennungssensor ergänzt. Dieser zusätzliche Gewebeimpedanzsensor besteht im allgemeinen aus einem Hilfsgenerator, der ein Signal mit einer Frequenz kleiner als die Arbeitsfrequenz des Hochfrequenz-Oszillators erzeugt. Statt eines Signales niedriger Frequenz kann auch eine Gleichspannung verwendet werden. Diese Niederfrequenz bzw. Gleichspannung wird in den Ausgangskreis des Hochfrequenz-Generators eingekoppelt. Die Bedämpfung des Niederfrequenz- bzw. Gleichspannungs-Hilfssignals ist nun ein Maß für die Kontaktqualität zwischen der Aktivelektrode und dem Gewebe bzw. zwischen der Aktivelektrode, dem Gewebe des Patienten und der Neutralelektrode. Da dieses Hilfssignal über die auch für die Abgabe der Hochfrequenzleistung erforderlichen Leitungen geführt wird, muß die Arbeits-/Nutzfrequenz des Hochfrequenzoszillators mittels einer geeigneten Filtereinrichtung abgeblockt werden. Mittels eines Schwellenwert-Schalters kann im einfachsten Fall ein Signal "Gewebekontakt vorhanden/nicht vorhanden" abgeleitet werden. Bevorzugt wird jedoch ein das Ausmaß des Gewebekontakts und die Größe der Gewebeimpedanz berücksichtigendes Signal gewonnen, mittels dessen die Regelung der übrigen Parameter optimiert werden kann.

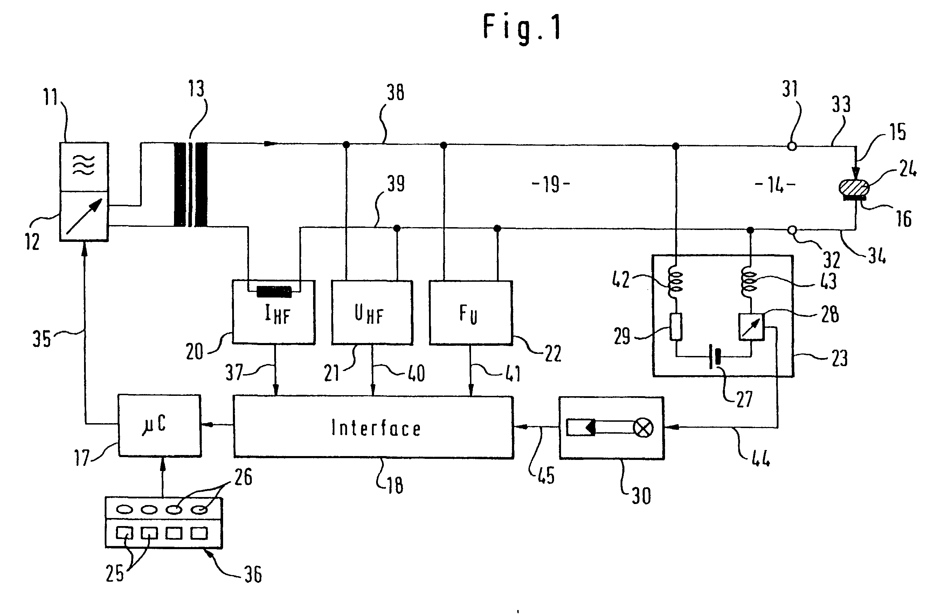

Die Erfindung wird im folgenden beispielsweise anhand der Zeichnung beschrieben; in dieser zeigt
- Figur 1: ein Blockschaltbild eines erfindungsgemäßen Hochfrequenz-Generators.

Nach Figur 1 weist ein Hochfrequenz-Generator einen Hochfrequenz-Oszillator 11 und einen von diesem gespeiste Leistungsstufe 12 auf, welche über einen Ausgangstransformator bzw. -übertrager 13 an einen Hochfrequenz-Ausgangskreis 19 angeschlossen ist, der zwei Ausgangsklemmen 31, 32 für den Anschluß einer Aktivelektrode 15 und einer Neutralelektrode 16 aufweist. Die Zuleitungen 33, 34, die daran angeschlossene Aktivelektrode 15 bzw. Neutralelektrode 16 und ein Patientenkörper 24, an den die Aktivelektrode 15 und die Neutralelektrode 16 angelegt sind, bilden zusammen einen Patientenstromkreis 14.

Die Leistungsstufe 12 des erfindungsgemäßen Hochfrequenz-Generators ist durch einen Mikrocomputer 17 steuerbar, mit welchem sie über eine oder mehrere Steuerleitungen 35 verbunden ist.

Der Mikrocomputer 17 erhält seine Eingangsinformationen von einer Tastatur 36 mit Funktionstasten 25, 26 sowie von einem Interface 18, welches Eingangssignale von einem in den Ausgangskreis 19 eingeschalteten Stromsensor 20, einem an den Ausgangskreis 19 angeschlossenen Spannungssensor 21 sowie einem ebenfalls an den Ausgangskreis 19 angeschlossenen Lichtbogensensor 22 erhält.

Der Stromsensor 20 wird vom im Ausgangskreis 19 fließenden Hochfrequenzstrom I_{HF} durchflossen und gibt über eine Verbindung 37 ein entsprechendes Signal an das Interface 18 ab.

Der Spannungssensor 21 ist an die beiden Ausgangsleitungen 38, 39 des Ausgangskreises 19 angeschlossen und gibt an das Interface 18 über eine Verbindung 40 ein der Ausgangs-Hochfrequenzspannung U_{HF} entsprechendes Signal ab.

Der Lichtbogensensor 22 mißt am Ausgangskreis 19 einen für das Auftreten von Funken oder eines Lichtbogens charakteristischen Parameter, z.B. Oberwellen oder bestimmte Gleichspannungskomponenten und gibt über eine Verbindung 41 ein Signal an das Interface 18 ab, welches ein Maß für das Nichtvorhandensein, Entstehen oder Vorhandensein eines Lichtbogens zwischen der Aktivelektrode 15 und dem Gewebe des Patientenkörpers 24 ist.

Erfindungsgemäß ist an den Ausgangskreis 19 des Hochfrequenz-Generators außerdem ein Gewebeimpedanzsensor 23 angeschlossen, der eine Gleich-Hilfsspannungsquelle 27 aufweist, die über einen Strombegrenzungswiderstand 29, eine Gleichstrom-Detektorschaltung 28 und Hochfrequenzfilter 42, 43 an die Ausgangsleitungen 38, 39 des Ausgangskreises 19 angeschlossen ist. Die Hilfsspannungsquelle 27 liefert in Zusammenwirkung mit dem Strombegrenzungwiderstand 29 einen Gleichstrom in den Patientenstromkreis 14, der eine für den Patientenkörper 24 unschädlichen Wert von 10 µA nicht übersteigen sollte.

Am Ausgang der Gleichstrom-Detektorschaltung 28 erscheint ein für den durch den Patientenstromkreis 14 fließenden Gleichstrom repräsentatives Signal, welches über eine Verbindung 44, eine galvanische Trennungsstufe 30, z.B. in Form eines Optokopplers und eine weitere Verbindung 45 ebenfalls in das Interface 18 eingespeist wird.

Die Funktion des beschriebenen Hochfrequenz-Generators ist wie folgt:

Durch Drücken bestimmter Funktionswahltasten 25, 26 auf der Tastatur 36 wird eine gewünschte Betriebsart des Hochfrequenz-Generatos angewählt, z.B. "Schneiden normal (unverschorft)", "Schneiden verschorft" oder "Koagulieren".

Anschließend wird dann die Neutralelektrode 16 an geeigneter Stelle des Patientenkörpers 24 angelegt bzw. befestigt und der Generator eingeschaltet. Nunmehr wird über den Ausgangsübertrager 13 eine Hochfrequenzspannung bzw. ein Hochfrequenzstrom an den Ausgangskreis 19 und den über die Ausgangsklemmen 31, 32 angeschlossenen Patientenstromkreis 14 angelegt. Dem Mikrocomputer 17 wird in jenem Augenblick durch den Stromsensor 20 der aktuelle Hochfrequenzstrom I_{HF}, über den Spannungssensor 21 die aktuelle Hochfrequenzausgangsspannung U_{HF} und über den Lichtbogensensor 22 ein Signal F_{U} übermittelt, welches für den Grad der Lichtbogenbildung repräsentativ ist.

Außerdem wird dem Mikrocomputer 17 vom Gewebeimpedanzsensor 23 der Gleichstrom-Übergangswiderstand zwischen der Aktivelektrode 15, dem Patientenkörper 24 und der Neutralelektrode 16 mitgeteilt, woraus der Mikrocomputer 17 erkennt, ob bereits ein Kontakt zwischen der Aktivelektrode 15 und dem Patientenkörper 24 bzw. zwischen dem Patientenkörper 24 und der Neutralelektrode 16 vorhanden ist oder nicht. Entsprechend steuert der Mikrocomputer 17 die Leistungsstufe 12 auf einen für den Operateur und den Patienten ungefährlichen Wert ein.

Auch während des Elektrochirurgie-Betriebes meldet der Gewebeimpedanzsensor 23 jeweils den aktuellen Gleichstromwiderstand im Patientenstromkreis 14 an den Mikrocomputer 17, so daß dieser neben dem Hochfrequenzstrom I_{HF}, der Hochfrequenzspannung U_{HF} und dem Lichtbogenbildungssignal F_{U} auch den Gleichspannungswiderstand im Patientenstromkreis 14 bei der Steuerung der Leistungsstufe 12 berücksichtigen kann.

Während bevorzugt sämtliche drei Sensoren 20, 21, 22 neben dem Gewebeimpedanzsensor 23 vorgesehen sind, ist es zumindest erforderlich, daß außer dem Gewebeimpedanzsensor 23 wenigstens noch der Lichtbogensensor 22 oder einer der beiden übrigen Sensoren 20, 21 vorgesehen ist.

In der Zeichnung ist die Hilfsspannungsquelle 27 als Gleichspannungsbatterie veranschaulicht. Sie kann aber auch durch ein Netzgerät mit Gleichrichtung oder eine niederfrequente Wechselspannungsquelle verwirklicht werden, wobei lediglich auf eine geeignete galvanische Entkopplung zwischen der Wechselspannungsseite des Netzgerätes und dem Ausgangskreis 19 zu achten ist.

Aufgrund der erfindungsgemäßen Schaltung kann auch bei sehr unterschiedlich geformten Aktivelektroden 15, welche das Behandlungsinstrument darstellen, automatisch jeweils eine optimale Hochfrequenz-Energiezufuhr gewährleistet werden.

### Bezugszeichenliste

- 11: Oszillator
- 12: Leistungsstufe
- 13: Ausgangstransformator
- 14: Patientenstromkreis
- 15: Aktivelektrode
- 16: Neutralelektrode
- 17: Mikrocomputer
- 18: Interface
- 19: Ausgangskreis
- 20: Stromsensor
- 21: Spannungssensor
- 22: Lichtbogensensor
- 23: Gewebeimpedanzsensor
- 24: Patientenkörper
- 25: Funktionswahltasten
- 26: Funktionswahltasten
- 27: Hilfsspannungsquelle
- 28: Detektorschaltung
- 29: Strombegrenzungswiderstand
- 30: galvanische Trennstufe
- 31: Ausgangsklemme
- 32: Ausgangsklemme
- 33: Zuleitung
- 34: Zuleitung
- 35: Steuerleitung
- 36: Tastatur
- 37: Verbindung
- 38: Ausgangsleitung
- 39: Ausgangsleitung
- 40: Verbindung
- 41: Verbindung
- 42: Hochfrequenzfilter
- 43: Hochfrequenzfilter
- 44: Verbindung
- 45: Verbindung

## Patentansprüche

1. Elektrochirurgischer Hochfrequenz-Generator mit einem Hochfrequenz-Oszillator (11) und einer an diesen angeschlossenen regelbaren Leistungsstufe (12), an die über einen Ausgangstransformator (13) ein Patientenstromkreis (14) mit Aktiv- und Neutralelektrode (15, 16) anschließbar ist, wobei an einem Ausgangskreis (19) des Generators mindestens ein Parametersensor (20, 21, 22) vorgesehen ist, der einen für die Patientenbehandlung charakteristischen Parameter, wie die Hochfrequenzspannung und/oder den Hochfrequenzstrom und/oder die Lichtbogenintensität erfaßt und ein solches Regelsignal für die Leistungsstufe (12) erzeugt, daß der Parameter je nach einer vorgegebenen Betriebsart auf bestimmten Werten oder in bestimmten Wertebereichen gehalten wird, wobei zusätzlich ein Gewebeimpedanzsensor (23) vorgesehen ist,
**dadurch gekennzeichnet,**
**daß** der Parametersensor (20, 21, 22) über ein Interface (18) an einen die Leistungsstufe (12) regelnden Mikrocomputer (17) angeschlossen ist,
**daß** der Gewebeimpedanzsensor (23) über das Interface (18) an den Mikrocomputer (17) angeschlossen ist,
**daß** für die Auswahl verschiedener Betriebsarten Funktionswahltasten (25, 26) vorgesehen sind, wobei mit dem Betätigen jeder Funktionstaste (25, 26) auch die Einflußnahme durch den Gewebeimpedanzsensor (23) ausgewählt ist, und
**daß** in einer Betriebsart "Schneiden mittels Funken bzw. Lichtbogen" die Leistungsregelung allein mittels des Gewebeimpedanzsensors (23) und des bzw. der übrigen Parametersensoren (20, 21, 22) ohne ein von außen bedienbares Leistungsstellglied erfolgt, wobei die Regelung aufgrund der Signale von dem Parametersensor (20, 21, 22) durch das Signal von dem Gewebeimpedanzsensor (23) so variiert wird, daß in jedem Augenblick das gerade vorhandene Maß an Gewebekontakt bzw. Stromleitfähigkeit berücksichtigt wird.

2. Hochfrequenz-Generator nach Anspruch 1,
**dadurch gekennzeichnet,**
**daß** das Signal von dem Gewebeimpedanzsensor (23) im Mikrocomputer (17) gegenüber den Signalen von dem Parametersensor (20, 21, 22) derart bevorzugt ist, daß letztere erst voll wirksam werden, wenn das Signal von dem Gewebeimpedanzsensor (23) den für eine einwandfreie Behandlung erforderlichen Gewebekontakt signalisiert.

3. Hochfrequenz-Generator nach Anspruch 1 oder 2,
**dadurch gekennzeichnet,**
**daß**
- im niederohmigen Bereich, d.h. bei Körperkontakt der Aktivelektrode (15) ein Signal "Körperkontakt vorhanden" und
- im hochohmigen Bereich, d.h. bei keinem Körperkontakt der Aktivelektrode (15), ein Signal "Kontakt nicht vorhanden" erzeugt wird und in letzterem Fall die übrigen Parameter, wie Hochfrequenzspannung und/oder Hochfrequenzstrom und/oder Hochfrequenzleistung auf ein sicheres Niveau heruntergeregelt werden.

4. Hochfrequenz-Generator nach einem der vorhergehenden Ansprüche,
**dadurch gekennzeichnet,**
**daß** der Gewebeimpedanzsensor (23) ein für das Ausmaß des Gewebekontaktes und die Stromleitfähigkeit des Gewebes repräsentatives analoges oder digitales Signal an den Mikrocomputer (17) liefert.

5. Hochfrequenz-Generator nach einem der vorhergehenden Ansprüche,
**dadurch gekennzeichnet,**
**daß** eine Betriebsart "Koagulieren" vorgesehen ist, und daß der Gewebeimpedanzsensor (23) auch zur Feststellung des optimalen Koagulationsgrades verwendet wird.

6. Hochfrequenz-Generator nach einem der vorhergehenden Ansprüche,
**dadurch gekennzeichnet,**
**daß** der Gewebeimpedanzsensor (23) ein Signal proportional dem Niederfrequenz- oder Gleichspannungs-Übergangswiderstand zwischen Aktivelektrode (15), Patientenkörper (24) und Neutralelektrode (16) liefert.

7. Hochfrequenz-Generator nach einem der vorhergehenden Ansprüche,
**dadurch gekennzeichnet,**
**daß** der Gewebeimpedanzsensor (23) aus einer an den Ausgangskreis (19) bzw. den Patientenstromkreis (14) angeschlossenen Hilfsspannungsquelle (27) mit einer niedriger als die Oszillatorfrequenz liegenden Frequenz und einer den von der Hilfsspannungsquelle (27) abgegebenen Strom erkennenden Detektorschaltung (28) besteht, welche über das Interface (18) an den Mikrocomputer (17) angeschlossen ist.

8. Hochfrequenz-Generator nach Anspruch 7,
**dadurch gekennzeichnet,**
**daß** zwischen die Hilfsspannungsquelle (27) und den Ausgangskreis (19) bzw. dem Patientenstromkreis (14) eine Strombegrenzungsanordnung (29) geschaltet ist, welche den von der Hilfsspannungsquelle (27) abgegebenen Strom auf einen für den Patientenkörper (24) unschädliches Maß von z.B. 5 µA bis 10 µA begrenzt.

9. Hochfrequenz-Generator nach Anspruch 7 oder 8,
**dadurch gekennzeichnet,**
**daß** zwischen die Detektorschaltung (28) und das Interface (18) eine galvanische Trennstufe (30) geschaltet ist.

10. Hochfrequenz-Generator nach einem der Ansprüche 7 bis 9,
**dadurch gekennzeichnet,**
**daß** die Frequenz der Hilfsspannungsquelle (27) eine Niederfrequenz von z.B. 10 bis 100 Hz, vorzugsweise 40 bis 60 Hz und insbesondere etwa 50 Hz ist.

11. Hochfrequenz-Generator nach einem der Ansprüche 7 bis 9,
**dadurch gekennzeichnet,**
**daß** die Hilfsspannungsquelle (27) eine Gleichspannungsquelle ist.

## Claims

1. Electrosurgical high frequency generator comprising a high frequency oscillator (11) and a regulatable power stage (12) connected to the latter, with a patient current circuit (14) with an active electrode and a neutral electrode (15 and 16) being connectable to the power stage (12) via an output transformer (13), wherein at least one parameter sensor (20, 21, 22) is provided at an output circuit (19) of the generator and detects a parameter characteristic for the treatment of the patient, such as the high frequency voltage and/or the high frequency current and/or the intensity of the arc and produces a regulating signal for the power stage (12) such that the parameter is kept at specific values or in specific value ranges in accordance with a preset mode of operation, wherein a tissue impedance sensor (23) is additionally provided
**characterised in that** the parameter sensor (20, 21, 22) is connected via an interface (18) to a microcomputer (17) which regulates the power stage (12);
**in that** function selection keys (25, 26) are provided for the selection of different operating modes, wherein, on actuating each function key (25, 26), the influence to be exerted by the tissue impedance sensor (23) is also selected; and
**in that** in an operating mode "cutting by means of sparks or arc", the power regulation takes place solely by means of the tissue impedance sensor (23) and of the remaining parameter sensor or sensors (20, 21, 22) without a power setting element operable from the outside, wherein the regulation is so varied as a result of the signals from the parameter sensor (20, 21, 22) by the signal from the tissue impedance sensor (23) that at any instant the degree of tissue contact or current conducting ability which is actually present is taken into account.

2. High frequency generator in accordance with claim 1,
**characterised in that** the signal from the tissue impedance sensor (23) is given preference in the microcomputer (17) relative to the signals from the parameter sensor (20, 21, 22) in such a way that the latter first become fully active when the signal from the tissue impedance sensor (23) signalises the tissue contact necessary for a problem-free treatment.

3. High frequency generator in accordance with claim 1 or claim 2,
**characterised in that**
- a signal "body contact present" is generated in the low ohmic region, i.e. on body contact of the active electrode (15) and
- a signal " contact not present" is generated in the high ohmic region, i.e. when no body contact with the active electrode (15) is present and, in the latter case, the remaining parameters, such as the high frequency voltage and/or the high frequency current and/or the high frequency power are regulated down to a safe level.

4. High frequency generator in accordance with any one of the preceding claims, **characterised in that** the tissue impedance sensor (23) delivers an analogue or digital signal to the microcomputer (17) representative of the degree of tissue contact and the current conducting ability of the tissue.

5. High frequency generator in accordance with any one of the preceding claims, **characterised in that** an operating mode "coagulation" is provided; and **in that** the tissue impedance sensor 23 is also used to determine the ideal degree of coagulation.

6. High frequency generator in accordance with any one of the preceding claims **characterised in that** the tissue impedance sensor (23) delivers a signal proportional to the low frequency or DC transition resistance between the active electrode (15), the patient's body (24) and the neutral electrode (16).

7. High frequency generator in accordance with any one of the preceding claims **characterised in that** the tissue impedance sensor (23) comprises an auxiliary voltage source (27) connected to the output circuit (19) or to the patient current circuit (14), and having a substantially lower frequency than the oscillator frequency, and a detector circuit (28) which recognises the current transmitted by the auxiliary voltage source (27) and which is connected via the interface (18) to the microcomputer (17).

8. High frequency generator in accordance with claim 7,
**characterised in that** a current limiting arrangement (29) is inserted between the auxiliary voltage source (27) and the output circuit (19) or the patient current circuit (14) and restricts the current transmitted from the auxiliary voltage source (27) to a level of for example 5 µA to 10 µA which is non-harming for the patient's body (24).

9. High frequency generator in accordance with claim 7 or claim 8, **characterised in that** a galvanic separating stage (30) is inserted between the detector circuit (28) and the interface (18).

10. High frequency generator in accordance with any one of claims 7 to 9, **characterised in that** the frequency of the auxiliary voltage source (27) is a low frequency of for example 10 to 100 Hz, preferably 40 to 60 Hz, and in particular of about 50 Hz.

11. High frequency generator in accordance with any one of claims 7 to 9,
**characterised in that** the auxiliary voltage source (27) is a DC voltage source.

## Revendications

1. Générateur de haute fréquence pour électrochirurgie, comportant un oscillateur à haute fréquence (11) et un étage de puissance réglable (12) raccordé à celui-ci, auquel peut être raccordé - via un transformateur de sortie (13) - un circuit électrique de patient (14) avec une électrode active et une électrode neutre (15, 16), au moins un détecteur de paramètre (20, 21, 22) étant prévu sur un circuit de sortie (19) du générateur, qui détecte des paramètres caractéristiques pour le traitement d'un patient, tels que la tension à haute fréquence et/ou le courant à haute fréquence et/ou l'intensité de l'arc lumineux, et qui génère un signal de régulation pour l'étage de puissance (12) tel que le paramètre est maintenu à des valeurs déterminées ou dans des plages de valeurs déterminées en fonction d'un mode de fonctionnement prédéterminé, et dans lequel est prévu en supplément un détecteur d'impédance tissulaire (23), **caractérisé en ce que** le détecteur de paramètre (20, 21, 22) est raccordé via une interface (18) à un micro-ordinateur (17) régulant l'étage de puissance (12), **en ce que** le détecteur d'impédance tissulaire (23) est raccordé via l'interface (18) au micro-ordinateur (17), **en ce que** des touches de sélection de fonction (25, 26) sont prévues pour le choix de modes de fonctionnement différents, et lors de l'actionnement de chaque touche de fonction (25, 26) l'influence sur le détecteur d'impédance tissulaire (23) est également sélectionnée, et **en ce que** dans un mode de fonctionnement "coupe par étincelles ou par arc lumineux", la régulation de puissance s'effectue uniquement au moyen du détecteur d'impédance tissulaire (23) et du ou des autres détecteurs de paramètres (20, 21, 22), sans organe de réglage de puissance manipulable depuis l'extérieur, la régulation étant variée en raison des signaux du détecteur de paramètre (20, 21, 22) par le signal du détecteur d'impédance tissulaire (23), de telle sorte que la valeur actuellement présente de contact tissulaire ou de conductivité électrique est prise en compte à chaque instant.

2. Générateur de haute fréquence selon la revendication 1, **caractérisé en ce que** le signal du détecteur d'impédance tissulaire (23) dans le micro-ordinateur (17) est préféré par rapport aux signaux du détecteur de paramètre (20, 21, 22), de telle sorte que ces derniers deviennent totalement efficaces uniquement lorsque le signal du détecteur d'impédance tissulaire (23) signalise le contact tissulaire requis pour un traitement parfait.

3. Générateur de haute fréquence selon l'une ou l'autre des revendications 1 et 2, **caractérisé en ce que**
- dans la plage de basse impédance, c'est-à-dire lors d'un contact de l'électrode active (15) avec le corps, un signal "présence de contact avec le corps" est généré, et
- dans la plage de haute impédance, c'est-à-dire lorsqu'il n'y a pas de contact de l'électrode active (15) avec le corps, un signal "absence de contact" est généré, et dans ce dernier cas, les autres paramètres, tels que la tension à haute fréquence et/ou le courant à haute fréquence et/ou la puissance à haute fréquence sont régulés à la baisse vers un niveau sûr.

4. Générateur de haute fréquence selon l'une des revendications précédentes, **caractérisé en ce que** le détecteur d'impédance tissulaire (23) fournit au micro-ordinateur (17) un signal analogique ou numérique représentatif de la valeur du contact tissulaire et de la conductivité électrique tissulaire.

5. Générateur de haute fréquence selon l'une des revendications dépendantes, **caractérisé en ce qu'**il est prévu un mode de fonctionnement "coagulation", et **en ce que** le détecteur d'impédance tissulaire (23) est utilisé également pour constater le degré de coagulation optimal.

6. Générateur de haute fréquence selon l'une des revendications dépendantes, **caractérisé en ce que** le détecteur d'impédance tissulaire (23) fournit un signal proportionnel à la résistance de transition à basse fréquence ou à tension continue entre l'électrode active (15), le corps du patient (24) et l'électrode neutre (16).

7. Générateur de haute fréquence selon l'une des revendications dépendantes, **caractérisé en ce que** le détecteur d'impédance tissulaire (23) est constitué par une source de tension auxiliaire (27) branchée au circuit de sortie (19) ou au circuit électrique du patient (14) et présentant une fréquence inférieure à la fréquence d'oscillateur, et par un circuit détecteur (28) qui reconnaît le courant fourni par la source de tension auxiliaire (27) et qui est raccordé via l'interface (18) au micro-ordinateur (17).

8. Générateur de haute fréquence selon la revendication 7, **caractérisé en ce qu'**un agencement limiteur de courant (29) est raccordé entre la source de tension auxiliaire (27) et le circuit de sortie (19) ou le circuit électrique du patient (14), lequel limite le courant fourni par la source de tension auxiliaire (27) à une valeur non nuisible pour le corps du patient (24) de par exemple 5µA à 10µA.

9. Générateur de haute fréquence selon l'une ou l'autre des revendications 7 et 8, **caractérisé en ce qu'**un étage de séparation galvanique (30) est branché entre le circuit détecteur (28) et l'interface (18).

10. Générateur de haute fréquence selon l'une des revendications 7 à 9, **caractérisé en ce que** la fréquence de la source de tension auxiliaire (27) est une basse fréquence par exemple de 10 à 100 Hz, de préférence de 40 à 60 Hz et en particulier d'environ 50 Hz.

11. Générateur de haute fréquence selon l'une des revendications 7 à 9, **caractérisé en ce que** la source de tension auxiliaire (27) est une source de tension continue.
